# EUROPEAN PATENT APPLICATION

(11) **EP 2 808 675 A1**
(43) Date of publication of application: **03.12.2014**
(21) Application number: 13002819.4
(22) Date of filing: 31.05.2013
(51) Int. Cl.: G01N 27/12

(54) **Integrated metal oxide chemical sensor**

(71) Applicant: Sensirion AG, 8712 Stäfa (CH)
(72) Inventor: von Waldkirch, Marc, 8053 Zürich (CH)
(74) Representative: Mirza, Akram Karim

(57) **Abstract**

A chemical sensor assembly for measuring the concentration of a gas analyte in a sample of air is described having on a common substrate (14) a first and a second sensor (10,30) each with at least one layer (11) of a metal oxide arranged between two electrodes (16) with the first and second sensor (10,30) having essentially identical dimensions and structure and with the second sensor (30) including an additional barrier (31, 11) for the gas analyte so that the second sensor (30) is essentially insensitive to the gas analyte and can be a reference sensor (30) for the first sensor (10).

## Description

### FIELD OF THE INVENTION

The present invention relates to an integrated chemical sensor, particularly a gas sensor, using metal oxide. The sensor is sufficiently small to be located within the exterior shell or housing of a portable electronic device such as a mobile phone, tablet and the like.

### BACKGROUND OF THE INVENTION

Portable or mobile devices originally introduced as mobile phones or electronic agendas become more and more ubiquitous. As the processing power of their internal processors grows and equally the bandwidth for communication with stationary processors, such portable devices take on more and more the role of multi-purpose tools available to consumers and specialist users alike.

It has been recognized that portable devices can benefit from the presence of sensors capable of providing a chemical analysis of materials brought into contact or the vicinity of the device. Whilst there are many possible applications for such sensors, it suffices to consider for example the analysis of air surrounding the portable device. Such an analysis can be useful for multiple purposes such as testing for hazardous gases, breath analysis for general medical purposes or driving fitness, and the like.

However integrating such a sensor within the narrow confines of a modern day portable device poses a significant technical challenge. Typically for such devices only a very limited volume is acceded to additional sensors outside the core functionality of the device such as wireless voice or data communication, display, speaker, processors and battery. This means that the real overall dimensions of the sensor, its associated circuitry for control and readout have to be within or close to the sub-millimeter range.

A sensor with these outer dimensions can only be manufactured, if the active structures, i.e. the size of the metal oxide film between electrodes, are reduced in length to below 100 microns or even below 50 microns or even less, as interface effects start to become more pronounced at these dimensions. However, in metal oxide sensors of this size the changes of electrical resistance caused by the gas become less whilst the resistance caused by interface effects between the (metallic) contact electrodes and the metal oxide film contribute in ever larger proportion to the measurement, thus making it more difficult to measure actual changes in gas concentrations.

It is therefore seen as an object of the invention to improve the chemical sensors using metal oxide films contacted through metallic electrodes, particularly for very small devices.

### SUMMARY OF THE INVENTION

Hence, according to a first aspect of the invention, there is provided a chemical sensor assembly for measuring the concentration of a gas analyte in a sample of air with the assembly comprising on a common substrate a first and a second sensor each comprising at least one layer of a metal oxide arranged between two electrodes with the first and second sensor having essentially identical dimensions and structure, with the second sensor being essentially insensitive to the gas analyte, for example by including an additional barrier for the gas analyte or delaying the response of the second sensor compared to the response of first sensor.

Sensors with essentially identical dimensions and structure are understood as being built so as to have in the absence of the analyte gas near identical responses to voltage and voltage variations as applied to the electrodes. It is preferred that the first and second sensor are manufactured basically according to the same specification and/or within the same batch manufacturing process to limit the variations between the two sensor to variations inherent in the manufacturing process and its tolerances with the exception of the means, such as a barrier, applied to reduce the amount of gas analyte reaching the active area. The active area is the area which contributes most to the sensor signal when the gas analyte penetrates the metal oxide layer.

Using the same batch process is seen as a way of ensuring that the important properties of the metal oxide layer such as grains size distribution, porosity and others are equal within the boundaries of statistical fluctuations.

A barrier can for example include a cap layer which is tight against the penetration of the gas analyte, or a layer of sufficient thickness to prevent at least most of the gas analyte to enter the active area and/or generate a significant signal change in the sensor. However, even if the barrier is not completely gas tight, the time to react to the change in the concentration of the gas can be much delayed between the two sensors. Hence it is still possible to use the second sensor during a limited initial period or phase of gas penetration as a reference sensor.

The sensor rendered essentially passive with regard to the gas analyte is used as a reference sensor for measurements conducted using the other sensor. It can be used to cancel out interface effects between electrode and MOX material. As the sensor size is reduced, for example to a length (as measured between the electrodes) of 100µm, 50µm or less, interface effects such as the Schottky barrier become a larger part of the signal. Hence their removal from the signal is of growing importance.

But a reference sensor as described herein can also be used to remove other parts of the measured signal as caused for example by contamination, general drift and aging effects, a temperature induced drift of the sensor signal and/or variations in the manufacturing process.

It can be advantageous to use more than one reference sensors insensitive to the gas to use for example averaged values in the compensation process.

In a particularly preferred variant of the invention, the sensors are linked to CMOS circuitry embedded within the same substrate. The CMOS circuitry can include adding/subtracting or similar signal processing elements using the reference signal as generated by the reference sensor to remove unwanted parts of the signal as measured by the other sensor.

In another preferred embodiment of the invention the substrate includes heating elements and the first and the second sensor are located at equivalent positions with regard to the heating elements so as to receive essentially the same amount of heat from them. In other words the first and second sensors are preferably located at interchangeable positions with respect to the heating elements exploiting for example a symmetry in the distribution of heating elements.

The metal oxide layer is best based on a metal-oxide such as tin oxide, tungsten oxide, gallium oxide, indium oxide, or zinc oxide in not-intentionally doped or doped form. The electrodes and or heating elements are preferably made of a metallic material, for example a noble metal.

In a preferred embodiment of the invention, a sensor in accordance with this invention is integrated as component within a portable electronic device having further uses other than chemical sensing. Enclosed in the housing are typically processors, drivers for parts such as screens, antennae, cameras, microphones and speakers as well as batteries to provide power to the device and its parts. A screen is typically arranged as a part of the housing or mounted behind a transparent window of the housing.

The above and other aspects of the present invention together with further advantageous embodiments and applications of the invention are described in further details in the following description and figures.

### BRIEF DESCRIPTION OF THE FIGURES

FIG. 1A is a schematic perspective view of a known metal oxide gas sensor;
FIG. 1B is schematic cross-section of the device of Fig. 1A;
FIG. 2 is an equivalent circuit diagram for a metal oxide gas sensor illustrating the effects of structures with reduced dimensions;
FIG. 3A is a schematic perspective view of a metal oxide gas sensor assembly in accordance with an example of the invention;
FIG. 3B is a schematic cross-section of a part of FIG. 3A;
FIG. 3C is a schematic cross-section of a variant of FIG. 3B; and
FIGs. 4A - 4B illustrate a device in accordance with an example of the invention in a mobile electronic device.

### DETAILED DESCRIPTION

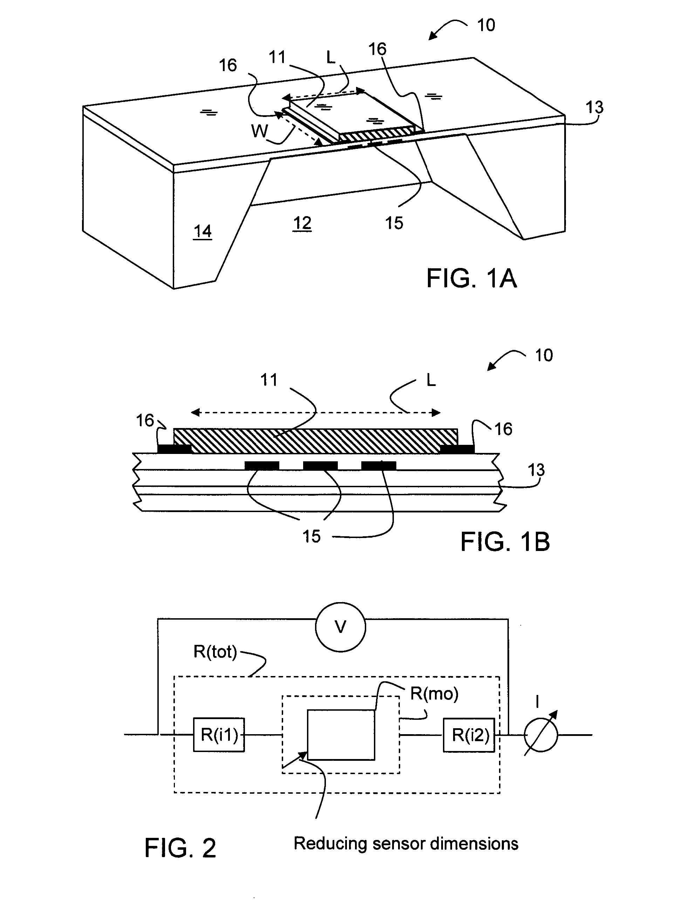

A gas sensor 10 with a sensing layer 11 of metal oxide is shown in FIGs. 1A and 1B. In these figures as in the ones following identical numerals are used to denote identical or similar features throughout the different embodiments.

The sensor is integrated with a CMOS circuitry (not shown) on a single chip. Parts of the CMOS layers 13 and handle layer 14 required for the CMOS circuit are etched away in a micro-mechanic (MEMS) step to form a cavity 12 at the location of the sensor. The remaining layers 13 form a thin membrane to support the sensing layer 11.

Embedded within the layers 13 the thin membrane are conducting elements 15 forming a heater to provide a local source of heat to heat the metal oxide 11 during operation of the sensor.

The metal oxide layer 11 is contacted by two conductive electrodes 16 and hence acts as a resistor. In the presence of an analyte this resistance changes thereby providing a measure of the concentration of the analyte in the immediate vicinity of the metal oxide layer.

The size or dimension which is of importance for this aspect of the invention is the length L of the sensor, which is the lateral extension of the metal oxide layer between the electrodes. For known sensors, this characteristic length is typically in the order of 100 microns or more. In FIG. 1 the dimension orthogonal to the length L is referred to as width W of the sensing layer or electrode.

In order to provide a higher integration and sensitivity within for example the limited confines and voltage supply of a portable electronic device such as a mobile phone, the characteristic length is advantageously reduced. Reducing the characteristic sizes of a sensor allows to manufacture smaller integrated sensors but also to place more sensor cells or pixels onto the same area.

However, the interface or Schottky effects increase with the attempted reduction in size. To illustrate such effects a total resistance R(tot) measured across the pair of electrodes 16 and the layer of metal oxide 11 can be represented as the sum of three resistors in series as shown in the equivalent circuit of FIG. 2. This equivalent circuit diagram emphasizes the contribution of the resistors R(i1) and R(i2) representing the (Schottky-)resistance at the interfaces between the metal oxide layer 11 and the two electrodes 16 to the total resistance R(tot).

In conventional metal oxide gas sensors the resistance R(mo) of the metal oxide layer is usually large and changes of it are readily accessible to the measurement without having regard to R(i1) and R(i2). However, the relative contribution of R(i1) and R(i2) to the measured total resistance R(tot) grows as the size of the sensor 10 is reduced. This effect caused by the reduction in the sensor dimensions is illustrated in FIG. 2 by the sizes of the two boxes R(mo) in dashed and solid line, respectively.

The size or dimension which is of importance for this aspect is the space between the electrodes 16, i.e. the inter-electrode distance, as bridged by the layer 11 of metal oxide and referred to in this specification as the length L of the sensor. Referring to the width of an electrode, i.e. its lateral extension, as W, the resistances R(i1) and R(i2) are proportional to 1/W, whereas the resistance R(mo) is proportional to the ratio L/W. The proportion R(i)/R(mo) between both types of resistances varies hence with 1/L, i.e. the relative importance of interface effects increases with decreasing electrode separation L. In case of meandering or other complex shaped electrodes the width is understood to be the minimal distance between the two electrodes. In the present example this characteristic dimension or width is assumed to be at least less than 100 microns, or less than 50 microns or even less than 30 microns.

As sensors become smaller it is therefore desirable to remove as much as possible of the resistances R(i1) and R(i2) and, in turn, increase the contribution of R(mo) to the measured resistance R(tot).

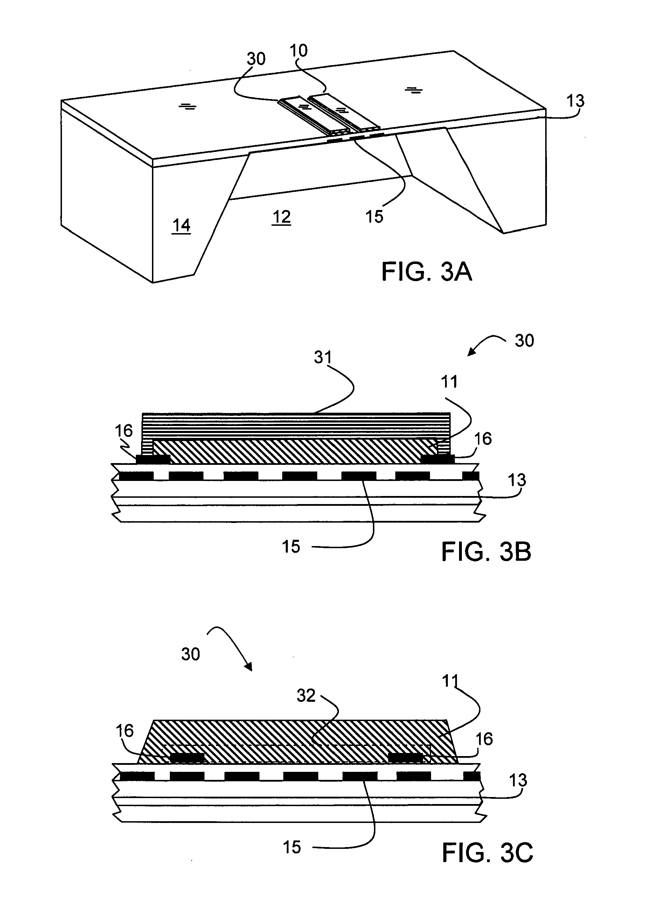

In the example shown in FIG. 3A a reference sensor 30 is placed next to the actual sensor 10. The sensor 30 is heated by the same heating elements 15 which are integrated into the membrane above the cavity 12. In the example the sensor 30 is manufactured during the same manufacturing process or run as the actual measuring sensor 10 and according to the same specification and using the same materials. Thus it is ensured that the sensors do not differ significantly in their internal structure.

It is worth noting that by using the same manufacturing process the metal oxide layer 11 of the reference sensor 30 has approximately the same microscopic structure as the metal oxide layer of the sensor 10. The grain structure, the grain boundaries and the porosity and their spatial distributions have an important influence on the measurement of an analyte gas. By reproducing these properties of the metal oxide layer in the reference sensor, it can be avoided that both sensors exhibit significantly different behavior after the manufacturing process.

The features of the reference sensor 30 and the measuring sensor 10 differ however in one important aspect. As shown in the cross-section of FIG. 3B which shows the reference sensor 30, the metal oxide layer is capped by a layer 31. The cap layer 31 prevents or at least impedes the penetration of the analyte into the deeper-lying MOX layer 11.

The cap layer 31 can be a non-porous layer protecting the MOX layer 11 from all gases. Such a layer can be manufactured using an additional step in the manufacturing process to deposit for example a silicon nitride (Si₃N₄). Silicon nitride can be deposited for example by using a LPCVD process through a mask as known as such. It is also possible to use other materials used in the electronics industry as passivation layers for the cap layer 31.

The use of a heteromaterial as the cap layer 31 can be avoided by increasing the thickness of the MOX layer 11 of the reference sensor 30 beyond the point where measureable amounts of the analyte gas can penetrate deep enough into the area between the electrodes 16 to cause a change in resistance. Such an embodiment of the invention is illustrated in FIG. 3C. The metal oxide layer 11 as shown embed the electrodes 16 such that the active area contributing the signal and shown surrounded by a dashed line 32 is effectively shielded from the analyte.

With the MOX layer 11 or at least its most active areas shielded from the gas analyte, the signals from the reference sensor 30 can be combined with signals from the measuring sensor 10 to enhance the signal relevant for the measurement, i.e. the resistance change caused by the analyte gas.

With the similarity in the interface structure between the two sensors, the contribution of the Schottky effect can be removed for example by subtracting the signal of the reference sensor 30 from the signal of the measurement sensor 10.

However, as metal oxide based sensors operate typically at elevated temperatures, e.g. between 200 and 500 degrees Celsius, it is advantageous to heat the reference sensor in the same manner as the measurement sensor. In the embodiments of FIGs. 3 both sensors are mounted above the heating elements 15 integrated into the membrane area of the substrate 14 above the cavity 12 and can thus be heated simultaneously and with essentially the same amount of heat. Using such an arrangement of the sensors, it is possible to remove also drifts caused by temperature from the measured signal in the same way as the Schottky effects. However it may be necessary to alter the heating between the two sensors to compensate for differences in their thermal behavior as caused by the slightly different structure.

The reference sensor can also be applied to detect and cancel long-term drifts in the performance of the sensor 10 by for example comparing the signal of both sensors from time to time in the absence of an analyte gas or any gases. Assuming the reference sensor 30 is not subjected to the same contamination as the measuring sensor 10, such repeated comparisons can be used to adjust the base line or otherwise recalibrate the sensor 10 increasing its useful lifetime.

A single reference sensor 30 can also serve as reference for several measuring sensors 10. For example a cluster of four sensors can be placed onto a membrane structure as shown in FIGs. 1 and 3 with one sensor being modified to serve as reference for the three other sensors.

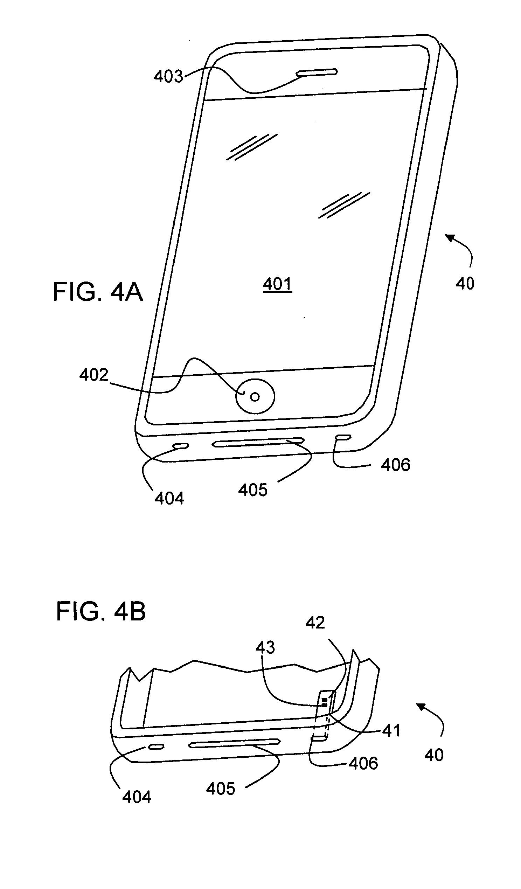

A chemical sensor in accordance with the above can be part of a portable electronic device such as a mobile phone as shown in FIG. 4A and 4B. As described above the chemical sensor needs to be of a (sufficiently small) size to fit within the limited volume available.

In FIG. 4A, the housing 40 of the mobile phone includes a front side with a screen 401 and elements like buttons 402 to let a user interact with the phone. Also shown on the front side is an opening 403 for a loudspeaker. Further openings 404,405 are located at a lower side wall of the housing 40. It is well known to mount components like microphones and loudspeakers behind such openings.

Another opening 406 is located at the lower side wall. As shown in FIG. 5B the opening 406 is linked to a tubular duct 41 passing through the interior of the housing. A chemical sensor 42 as described above and a humidity sensor 43 are both mounted along the duct 41 such that the sensitive areas of both sensors are essentially exposed air of the same composition entering the duct through the opening 406. The actual size and shape of the duct 41 depends on the volume available and the nature of the chemical sensor 42 and the humidity sensor 43, but given the physical constraints of portable mobile devices the diameter of the opening is typically in the range of less than 2mm and in the present example actually about 1mm.

The humidity sensors 43 can be manufactured as described for example in the international applications WO 2012/100362 or WO 96/19563. The humidity sensor is best combined with a temperature sensor. Such sensors are commercially available, e.g. from Sensirion™ under the trade name SHTC1. Both sensors are mounted adjacent to each other in the duct 41.

While there are shown and described presently preferred embodiments of the invention, it is to be understood that the invention is not limited thereto but may be otherwise variously embodied and practised within the scope of the following claims.

## Claims

1. A chemical sensor assembly for measuring the concentration of a gas analyte in a sample of air, the assembly comprising on a common substrate (14) a first and a second sensor (10,30) each comprising at least one layer (11) of a metal oxide arranged between two electrodes (16) with the first and second sensor (10,30) having essentially identical dimensions and structure and with the second sensor (30) being essentially insensitive to the gas analyte.

2. The chemical sensor assembly according to claim 1, comprising an additional barrier (31, 11) for the gas analyte.

3. The chemical sensor assembly according to claim 2, wherein barrier (31, 11) includes a cap layer preventing the penetration of the gas analyte.

4. The chemical sensor assembly according to claim 2 or 3, wherein barrier (31, 11) includes a layer of sufficient thickness to prevent at least most of the gas analyte to enter an active area located between the electrodes (16) of the second sensor (30).

5. The chemical sensor assembly according to claim 4, wherein barrier is part of the at least one layer (11) of a metal oxide increased in thickness compared to the corresponding at least one layer (11) of a metal oxide of the first sensor (10).

6. The chemical sensor assembly according to any of the preceding claims, wherein the at least one layer (11) of a metal oxide and the two electrodes (16) of the first and second sensor (10,30) are manufactured according to the essentially identical specifications and materials.

7. The chemical sensor assembly according to any of the preceding claims, wherein the substrate (14) includes heating elements (15) in a MEMS-type structure located below the first and the second sensor (10,30).

8. The chemical sensor assembly according to any of the preceding claims, wherein the second sensor (30) is a reference sensor for the first sensor (10) to remove unwanted parts including electrode interface effects from the signal as measured by the first sensor (10).

9. The chemical sensor assembly according to any of the preceding claims, wherein the substrate includes CMOS circuitry with contacts to the first and second sensor (10,30) and processing elements using signals from the second sensor (30) to remove unwanted parts including electrode interface effects from the signal as measured by the first sensor (10).

10. A portable electronic device comprising a chemical sensor assembly in accordance with any of the preceding claims.
